# EUROPEAN PATENT SPECIFICATION

(11) **EP 3 473 176 B1**
(45) Date of publication and mention of the grant of the patent: **16.09.2026**
(21) Application number: 18201550.3
(22) Date of filing: 19.10.2018
(51) Int. Cl.: A61B 5/296, A61B 5/389

(54) **ENDOTRACHEAL TUBE FOR MONITORING THE LARYNGEAL MUSCLES AND NERVES**
ENDOTRACHEALTUBUS ZUR ÜBERWACHUNG DER KEHLKOPFMUSKULATUR UND -NERVEN
TUBE ENDOTRACHÉAL POUR LE MONITORING DES NERFS ET MUSCLES LARYNGÉS

(30) Priority: 20.10.2017 EP 17306450
(43) Date of publication of application: 24.04.2019
(73) Proprietor: COMEPA-INDUSTRIES, 93170 Bagnolet (FR)
(72) Inventor: PELLERIN DE BEAUVAIS, Gilles, Hong-Kong (HK)
(74) Representative: Icosa

(56) References cited:
- EP-A1- 0 438 863
- WO-A2-2011/041684

## Description

### FIELD OF INVENTION

The present invention pertains to the field of medical devices. In particular, the invention relates to airway devices, such as tracheal tubes.

### BACKGROUND OF INVENTION

Tracheal tubes are often placed in the airway of a patient in medical situations that necessitate protection of the airway from possible obstruction or occlusion. For instance, tracheal tubes may be used in emergency situations, such as when a patient experiences cardiac or respiratory arrest.

Endotracheal tubes may include electrodes that are designed to make contact with a patient's vocal cords and perform laryngeal nerve and/or muscles monitoring. This configuration of endotracheal tubes is generally used to facilitate electromyographic (EMG) monitoring of the vocal cords during surgery when connected to an electromyographic monitoring device. Endotracheal tubes provide an open airway for patient ventilation, and provide for monitoring of electromyographic activity of the intrinsic laryngeal musculature when connected to an appropriate electromyographic monitor. Endotracheal tubes can provide continuous monitoring of the nerves supplying the laryngeal musculature during surgical procedures. A common procedure in which laryngeal EMG is used is a thyroid surgery.

An example of such an endotracheal tube for detecting clectromyographic signals in the laryngeal muscles and comprising electrode wires is disclosed in U.S. Patent 5,125,406 to Goldstone et al. This US patent discloses the use of electrically conductive metallic lead as electrodes positioned on the exterior surface of the endotracheal tube. This electrode configuration has a poor compacity generating sharp edges on the external surface of the endotracheal tube.

WO2011/041684A2 discloses an endotracheal tube apparatuses designed for EMG monitoring of laryngeal muscles, particularly using conductive ink electrodes on the tube's exterior surface (printed conductive ink electrodes, e.g., silver/carbon-based, their placement, longitudinal, circumferential, or on cuffs, and designs to reduce rotational sensitivity of the measured signals) to detect EMG signals from vocal folds during surgery.

With respect to the endotracheal tubes disclosed in the prior art, there is the necessity to further improve the quality of the signal retrieved (i.e. higher conductivity) while gather the overall dimensions of the electrodes as compact as possible to facilitate the insertion in the subject larynx and guarantee the bio-compatibility of the electrodes.

### SUMMARY

The invention is defined by the appended claims. One aspect of the present disclosure relates to a device for monitoring electromyographic signals of a subject's muscles and nerves comprising:
- a tube extending along a longitudinal axis and having an exterior surface and an internal surface; and
- at least two conductive electrodes coupled to the external surface of the tube, wherein the at least two conductive electrodes comprise a conductive layer deposited on a flexible polymer layer and are configured to receive electromyographic signals from muscles and nerves of the subject.

Another aspect of the present disclosure relates to a device for monitoring electromyographic signals of a subject's laryngeal muscles and laryngeal nerves comprising:
- an endotracheal tube extending along a longitudinal axis and having an exterior surface and an internal surface; and
- at least two conductive electrodes coupled to the external surface of the endotracheal tube and configured to receive electromyographic signals from laryngeal muscles and nerves when the endotracheal tube is placed in the trachea of the subject.

Yet another aspect of the present disclosure relates to a device for monitoring electromyographic signals of a subject's laryngeal muscles and laryngeal nerves comprising:
- an endotracheal tube extending along a longitudinal axis and having an exterior surface and an internal surface;
- at least two conductive electrodes coupled to the external surface of the endotracheal tube, wherein the at least two conductive electrodes comprise a conductive layer deposited on a flexible polymer layer and are configured to receive electromyographic signals from laryngeal muscles and nerves when the endotracheal tube is placed in the trachea of the subject.

The combination of a conductive layer and a flexible polymer layer, where the conductive layer is deposited on the flexible polymer layer, has the advantage of allowing the production of conductive electrodes with high conductivity and excellent flexibility in an extremely compact thickness.

According to one embodiment, the conductive layer of each conductive electrode is a conductive ink, notably a silver ink.

According to one embodiment, the device further comprises an adhesive coating to couple each conductive electrode to the endotracheal tube.

According to one embodiment, the at least two conductive electrodes, extending along the longitudinal axis, are laterally offset and are transversally aligned.

According to one embodiment, the endotracheal tube comprises a distal portion to be inserted into the subject's larynx and the endotracheal tube comprises an inflatable cuff at said distal portion.

According to one embodiment, the at least two conductive electrodes are spaced from the inflatable cuff of about 0.2 cm to 4 cm along the longitudinal axis.

According to one embodiment, the device further comprises at least two cables coupled to the at least two conductive electrodes and configured to carry the electromyographic signals received by the at least two conductive electrodes to a processing apparatus.

According to one embodiment, the endotracheal tube comprises an internal channel between the external surface and the internal surface, extending along the longitudinal axis of the endotracheal tube and being configured to lodge at least partially the at least two cables.

According to one embodiment, the endotracheal tube is a braided or reinforced endotracheal tube.

According to one embodiment, the device comprises at least four, six or eight conductive electrodes coupled to the external surface of the endotracheal tube.

According to one embodiment, the at least two conductive electrodes are regularly distributed (or regularly offset) in a direction transversal to the longitudinal axis so as to surround a circumference of the endotracheal tube.

According to one embodiment, the distal portion of the endotracheal tube comprises a Murphy's eye.

According to one embodiment, the device further comprises a connector configured to be mechanically connected to the proximal end of the endotracheal tube so as to couple the endotracheal tube to a mechanical ventilator.

According to one embodiment, each cable comprises a first portion extending into the internal channel and a second portion extending on the external surface of the endotracheal tube in contact with one electrode.

According to one embodiment, the second portion of the cables is covered by a non-conductive isolating covering.

### DEFINITIONS

In the present invention, the following terms have the following meanings:
- "As used herein the singular forms **"a", "an",** and **"the"** include plural reference unless the context clearly dictates otherwise.
- The term **"about"** is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent, preferably of 5 percent.
- **"Electrode"** refers to a conductor used to establish electrical contact with a nonmetallic part of a circuit, preferably a subject body. For instance, EEG electrodes are small metal discs usually made of stainless steel, tin, gold, silver covered with a silver chloride coating; there are placed on the scalp at specific positions.
- **"Electromyogram"** refers to the record of the electrical activity of a muscle or a nerve of a subject, made by electrodes applied on the subject's tissues.
- **"Ink"** refers to is a liquid or paste having a viscosity ranging from 0.0009 Pa • s to 200 Pa • s with a shear rate ranging from 05 to 1400000 1/s.
- **"Subject"** refers to a mammal, preferably a human. In the sense of the present invention, a subject may be a patient, i.e. a person receiving medical attention, undergoing or having underwent a medical treatment, or monitored for the development of a disease.

### BRIEF DESCRIPTION OF THE DRAWINGS

Features and advantages of the invention will become apparent from the following description of embodiments of a device according to the invention, this description being given merely by way of example and with reference to the appended drawings in which:
**Figure 1** shows an endotracheal tube comprising two electrodes on the external surface of the endotracheal tube according to a first embodiment.
**Figure 2** shows an endotracheal tube comprising four electrodes on the external surface of the endotracheal tube according to a second embodiment.
**Figure 3** shows a cross section of the endotracheal tube of Figure 1 or 2 comprising an internal channel and a lumen according to one embodiment.
**Figure 4** shows an open-up view of the four electrodes and the cables on the external surface of the endotracheal tube of Figure 2.
**Figure 5** shows an open-up view of the two electrodes and the cables on the external surface of the endotracheal tube of Figure 1.
**Figure 6** shows a cross section of an endotracheal tube comprising, on the external surface, a support on which are stuck the electrodes, according to a third embodiment.

### REFERENCE

1 - Device;
2 - Endotracheal tube;
3 - Conductive electrodes;
4 - Inflatable cuff;
5 - Cables;
6 - Internal channel;
7 - Lumen;
8 - Murphy's eye;
9 - Connector;
10 - Support;
20 - External surface;
21 - Internal surface.

### DETAILED DESCRIPTION

The following detailed description will be better understood when read in conjunction with the drawings. For the purpose of illustrating, the device is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

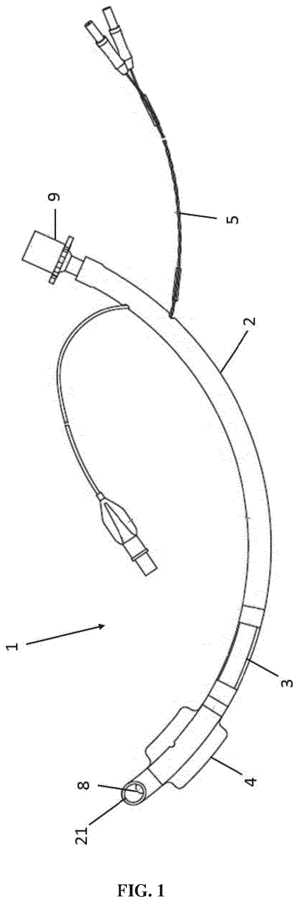

The present invention relates to a device **1** for monitoring electromyographic signals of a subject's laryngeal muscles and laryngeal nerves. With reference to Figure **1****,** said device **1** comprises an endotracheal tube **2** coupled to at least two conductive electrodes **3.**

It should be noted that the provided endotracheal tube may be used in conjunction with auxiliary devices, such as airway accessories, ventilators, humidifiers, and so forth, which may cooperate with the endotracheal tube to maintain airflow to and from the lungs of the subject. For further example, the endotracheal tubes may be coupled to an interface circuit and/or a monitor that is configured to receive data from the conductive electrodes, process such data, and display the processed data to an end user (e.g., medical technician, doctor, nurse, etc.).

In general, a conductive electrode, when in monitoring proximity to a target muscle or nerve, can measure activity of a muscle by simply detecting activity, or by detecting and measuring the level of activity against a predetermined value of activity. As used herein, monitoring proximity means that the electrode is close enough to the target muscle or nerve to detect that the muscle or nerve has been stimulated, or is undergoing some type of activity in response to a stimulator probe or other stimulation, such as for example a mechanical manipulation. For example, monitoring proximity can mean that the conductive electrode is close enough to the target muscle to detect electrical stimulation of the muscle.

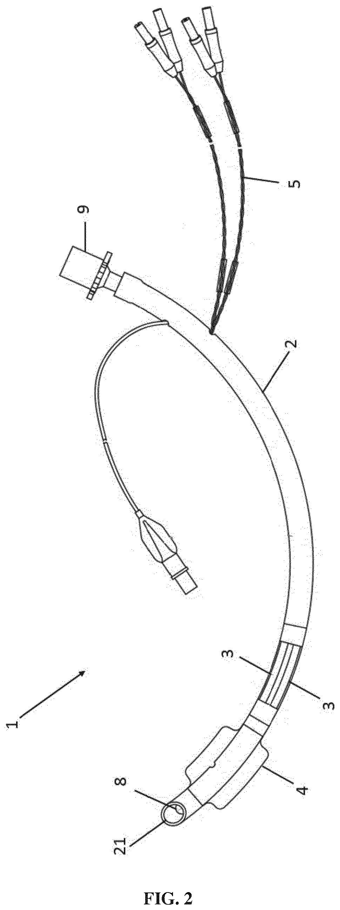

With reference to the embodiments represented in Figures 1 to 2, the endotracheal tube 2 extends along a longitudinal axis and comprises at least 2 electrodes 3 for collecting electromyographic signal.

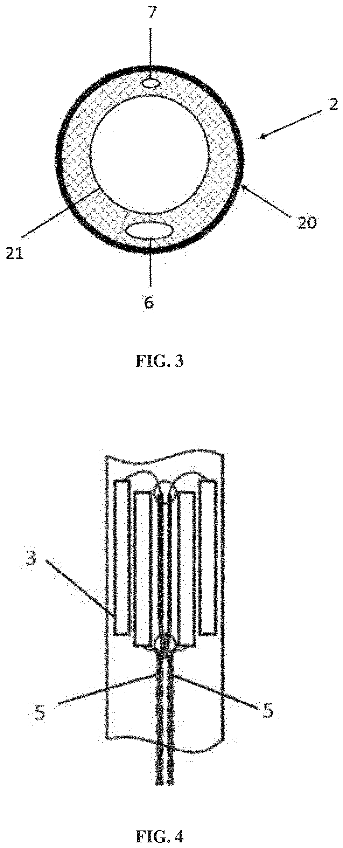

According to one embodiment, the endotracheal tube **2** extends along a longitudinal axis and has an exterior surface **20** and an internal surface **21,** as shown in Figure **3****.** According to one embodiment, the endotracheal tube **2** comprises a distal portion configured to be inserted into the subject trachea. There are many types of such endotracheal tubes. For example, endotracheal tubes range in size from 2 mm to 10.5 mm in internal diameter. Different sizes of tubes are chosen based on the patient's body size with the smaller sizes being used for pediatric and neonatal patients. Preferred sizes ranges from 5 mm to 8 mm or 4 mm to 9 mm.

According to one embodiment, the endotracheal tube **2** comprises or is made of polyvinyl chloride. Preferred materials for construction of the endotracheal tube are PVC or silicone polymers, such as Silastic^{®}. In one embodiment, the endotracheal tube **2** is a braided tube that is more flexible than conventional solid polymer tubes. According to one embodiment, the endotracheal tube **2** is formed from a braided polymer or nitinol (i.e. shape memory alloy) within a thin-walled tube, and reduces or eliminates rotation of the endotracheal tube at the vocal folds, while allowing a proximal portion of the endotracheal tube to rotate. According to an alternative embodiment, the endotracheal tube **2** is a spring coil reinforced tube. However, one skilled in the art will recognize that numerous alternative materials may be used to construct the endotracheal tube **2.**

According to one embodiment, the external surface **20** of the endotracheal tube **2** comprises tube placement markings in order to guide the surgeon during tube insertion. Said tube placement markings may be graduated markings, triangular marking, x-shape marking, numerical marking, color marking or the like.

According to one embodiment, the endotracheal tube **2** comprises an internal channel **6** arranged inside the internal volume of the endotracheal tube **2** defined by the external surface of the endotracheal tube **2.** According to one embodiment, the internal channel **6** is comprised between the external surface **20** and the internal surface **21,** extending along the longitudinal axis of the endotracheal tube **2.**

According to one embodiment, the endotracheal tube **2** comprises an inflatable cuff **4** at the endotracheal tube distal portion in order to seal the trachea and bronchial tree against air leakage and aspiration of gastric contents, blood, secretions, and other fluids.

According to one embodiment, the endotracheal tube **2** is an uncuffed endotracheal tube. According to one embodiment, the endotracheal tube **2** comprises a cuff inflating conduit configured to be connected to a source of injected air (not shown) for inflating the inflatable cuff **4.** Said cuff inflating conduit communicates with a lumen **7** located in the wall of tube, and the lumen **7** communicates with the inflatable cuff **4.**

According to one embodiment, the distal portion of the endotracheal tube **2** comprises a Murphy's eye **8** to prevent airway occlusion when the tube endotracheal tube **2** is improperly placed within the trachea. According to an alternative embodiment, the distal portion of the endotracheal tube **2** does not comprise a Murphy's eye.

According to one embodiment, the endotracheal tube **2** comprises a connector **9** configured to be mechanically connected to the proximal end of the endotracheal tube **2** to couple the endotracheal tube **2** to a mechanical ventilator.

According to one embodiment, the at least two conductive electrodes **3** are coupled to the external surface of the endotracheal tube **2.**

According to one embodiment, the at least two conductive electrodes **3** each comprise a conductive layer formed by a conductive ink deposited on a flexible polymer layer and are configured to receive electromyographic signals from laryngeal muscles and nerves when the endotracheal tube is placed in the trachea of the subject. The conductive ink may comprise a mixture of conductive materials, preferably metallic particles or metallic salts or oxides dissolved or suspended in a liquid carrier. Suitable conductive particles include, but are not limited to, finely divided particles or flakes of silver, silver salts such as silver chloride, silver oxide, gold, copper, copper chloride, copper, platinum, carbon or graphite. The conductive materials may be provided in the form of pellets, flakes, and nanoparticles.

According to a preferred embodiment, the conductive ink of the conductive electrodes 3 is a silver ink. Silver is preferred as conductive material because it is highly conductive (it has a resistivity of 1.62x10⁻⁸ Ω•m), is oxidatively stable and has the added advantage that its oxide is electrically conductive. The thickness of the silver ink deposited on the flexible polymer layer may be comprised between 10 µm and 500 µm. According to one embodiment, the thickness of the silver ink may range from 10 µm and 100 µm, said reduced thickness has the advantage to contribute to the production of light and compact electrodes. According to one embodiment, the thickness of the silver ink may range from 101 µm and 500 µm, said thickness range has the advantage to contribute to the production of highly conductive electrodes allowing to obtain a good quality signal.

Numerous suitable conductive inks are available from CSC (Chang Sung Corporate), such as silver paste, product number Paron-910A.

The silver ink typically has a viscosity in excess of 5 Pa • s, preferably about 20 - 30 Pa • s. The viscosity should also be such that the conductive ink once deposited on the flexible polymer layer does not flow so that the dimensions of the printed conductive electrode can be held with design parameters as such as the thickness.

As a variant, the conductive layer may be a layer deposited by conventional thin-film deposition processes such as, by way of nonlimiting examples: sputtering, in particular magnetron sputtering; chemical vapor deposition (CVD), in particular plasma-enhanced chemical vapor deposition (PECVD).

According to one embodiment, the flexible polymer layer is a carbon-based polymer layer. Examples of suitable polymers include, in particular, polyethylene terephthalate (PET), polyethylene naphthalate (PEN), polycarbonate, polyurethane, polymethyl methacrylate, polyamides, polyimides.

According to one embodiment, the flexible carbon-based polymer layer is a polyethylene-based film of thickness ranging from 0.005 to 1 mm.

Conductive inks can be deposited on the flexible polymer layer using various known technologies such as PAD printing, Screen printing, Ink jet dispensing, digital printing, Micropen dispensing, painting, vapor deposition and plasma sputtering.

The advantage of combining a silver conductive ink deposited on a flexible polymer layer, according to the embodiment hereabove, is to obtain conductive electrodes with high conductivity and excellent flexibility in an extremely compact thickness.

According to a preferred embodiment, the conductive electrodes **3** have a thickness ranging from 0.006 mm to 1.5 mm.

According to a preferred embodiment, the conductive electrodes **3** thickness is about 0,095 mm so that when the conductive electrodes **3** are coupled to the endotracheal tube **2,** the external diameter of the endotracheal tube **2** is substantially unchanged.

According to one embodiment, the device **1** further comprises an adhesive coating to couple the at least two conductive electrodes **3** to the endotracheal tube **2.** The adhesive coating may be in the form of a glue, a cement or a paste. The adhesive coating may be a solvent free acrylic adhesive, a polyester resin, polyols or acrylic polymers and the like. Preferably, adhesive coating may comprise an acrylic ester copolymer, a cross linker and/or a tackifier. The adhesive coating can be deposited on the surface of flexible polymer layer which is not coated with the conductive layer.

According to one embodiment, each conductive electrode **3** is formed by depositing the conductive layer on the flexible polymer layer prior to coupling with the endotracheal tube **2.**

Generally, without any modification, these types of conductive electrodes are fixed. According to one embodiment, the at least two conductive electrodes are immoveable relative to the endotracheal tube **2.**

According to one embodiment, the at least two conductive electrodes **3** extends along the longitudinal axis, are laterally offset and are transversally aligned. According to an alternative embodiment, the at least two conductive electrodes **3** are laterally offset and are not aligned transversally.

According to one embodiment, the distance between the at least two conductive electrodes **3** and the inflatable cuff **4** ranges from about 0.2 cm to about 4 cm along a longitudinal axis, so that when the endotracheal tube **2** is placed in the trachea of the subject the at least two conductive electrodes **3** may be in proximity of the nerve and/or muscles of the larynx.

According to one embodiment, the at least two conductive electrodes **3** are regularly offset on a direction transversal to the longitudinal axis to surround a circumference of the endotracheal tube **2.**

According to one embodiment, four conductive electrodes **3** are coupled to the external surface **20** of the endotracheal tube **2,** to provide a dual channel structure.

According to one embedment, six conductive electrodes **3** are coupled to the external surface **20** of the endotracheal tube **2,** to provide a tri channel structure. According to one alternative embedment, the external surface **20** of the endotracheal tube comprises 8 conductive electrodes **3.**

According to one embodiment, the conductive electrodes have a longitudinal dimension ranging from 0.5 cm to 5 cm. According to a preferred embodiment, longitudinal dimension ranges between 0.5 and 3 cm to have the best compromise for the contact area between the electrodes and laryngeal muscles/nerves to ensure a good signal intensity without collecting to much noise. According to one embodiment, the conductive electrodes have a lateral dimension ranging from 1 mm to 2 cm, depending on the size of the endotracheal tube and on the number of electrodes, the lateral dimension is chosen to avoid any lateral overlapping of the conductive electrodes.

According to one embodiment, the endotracheal tube **2** comprises at least two cables **5** coupled to the at least two conductive electrodes **3** and configured to carry the electromyographic signals received by the at least two conductive electrodes **3** to a processing apparatus.

According to one embodiment, the endotracheal tube **2** comprises an internal channel **6** which is configured to lodge at least partially the at least two cables **5.**

According to the embodiment wherein the endotracheal tube comprises four conductive electrodes, the cables **5** coupled to the conductive electrodes **3** are at least four. The cables **5** may be of equal number of the conductive electrodes **3** coupled to the external surface of the endotracheal tube **2.**

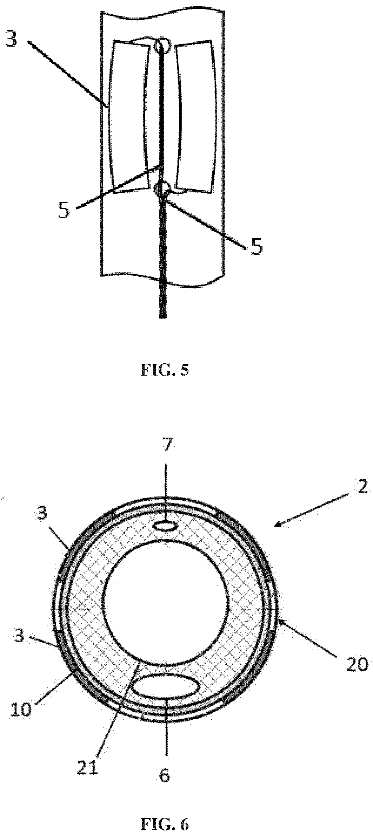

According to one embodiment, each cable **5** comprises a first portion extending into the internal channel **6** and a second portion extending in the external surface **20** of the endotracheal tube **2** in contact with one electrode **3.** According to one embodiment, the internal channel **6** comprises at least a through hollow, preferably two through hollows, in proximity of the conductive electrodes **3** which is configured to take the cables **5** in contact with the conductive electrodes **3** on the external surface of the endotracheal tube **2,** as shown in Figure **4** and **5****.**

According to one embodiment, the second portion of the cables **5** is covered by a non-conductive isolation covering. Said second portions may be covered by non-conductive insolating covering for example made of PVC, silicon or the like. Said insulating covering may be also used to ensure the contact between the cables **5** and the conductive electrodes **3.** The advantage of collecting the electromyographic signals from the nerve and/or muscles by putting the cables **5** in direct contact with the conductive electrodes **3** is a reduced signal to noise ratio, improving the nerve monitoring efficiency.

According to one embodiment represented in Figure 6, the device **1** further comprises a support **10** in contact with the external surface of the endotracheal tube **2.** In this embodiment, the support **10** is a sheet of cellular or porous material, such as a foam, wrapped around the endotracheal tube **2** at least partially covering the external surface **20** so that the support **10** covers the whole circumference of the endotracheal tube **20** on a predefined length. The surface of the support **10** facing the external surface **20** may be stuck to the endotracheal tube **2.** In one embodiment, the conductive electrodes **3** are entirely placed on the support **10.** The conductive electrodes **3** may be stuck to the support **10** by means of an adhesive coating. The thickness of the support **10** may range from 0.1 mm to 2 mm, notably range from 0.2 mm to 0.7 mm so that the support is thin enough to minimize dimensions of the medical device **1.**

In one embodiment, the support **10** is a flexible, extensible and/or deformable substrate. In one embodiment, the support **10** is a polymer material, notably a polystyrene or a polyester. According to a preferred embodiment, the support **10** is a flexible, extensible and longitudinally deformable foam of polymer material such as polystyrene or polyester.

Such a support **10** acts as a damper, advantageously allowing to reduce traumatisms of the vocal cords when the device **1** is introduced in the trachea of the subject.

According to an alternative embodiment, the electrodes are wirelessly connected to the processing apparatus.

Prior and currently available devices include sensors and cables on the surface of the endotracheal tube which are raised and can have sharp edges and which can cause injury to the tissue of the throat, larynx and vocal cords. The advantage of the currently described device with cables passing directly inside the endotracheal tube thickness the endotracheal tube is to have an external surface smooth where these hazards are eliminated.

While the device described herein is an endotracheal tube, the man skilled in the art will also recognize that other medical devices, such as catheters can be prepared in the same manner to have conductive electrodes on the surface thereof.

According to one embodiment, the device **1** also includes an output system, which can be in communication, either via a direct electrical wire or wirelessly, with the conductive electrodes **3.** In general, the output system may comprise an external electromyographic monitoring device that provides output indicative of measured activity of the target muscle and/or the target nerve when the endotracheal tube **2** is positioned adjacent or on the target nerve. The output system can include a controller or the processing apparatus that receives signals or data from the at least two electrodes **3,** processes the signals, and outputs information or an alarm to a health care provider as to the sensed muscle activity, and thus the proximity of the at least two electrodes **3** to the target nerve. The output of the output system can be an audible alarm from a speaker, and/or a visual alarm via a light or screen.

An example of device according to the embodiment here above may comprise a PVC cuffed endotracheal tube extending along a longitudinal axis and two conductive electrodes coupled to the external surface of the endotracheal tube. The two conductive electrodes may extend along the longitudinal axis, laterally offset and transversally aligned. These two conductive electrodes may comprise a 0.005 mm layer of silver conductive ink deposited on a flexible polymer layer of 0.07 mm thickness. Such conductive electrodes present a resistivity lower that 10 Ohm/sqm (at 24 °C), ensuring a good conductivity of the signal. The conductive electrodes may have a longitudinal dimension of about 2 cm and a lateral dimension of about 0.5 cm and be spaced of about 1 cm from the endotracheal tube inflatable cuff. Two cables may be coupled to the two conductive electrodes and configured to carry the electromyographic signals received by the two conductive electrodes to a processing apparatus. The two cables are partially lodge in an internal channel located in the wall of the endotracheal tube and arrive on the external surface of the endotracheal tube passing through two through hollows so as to be in contact to the surface of the conductive electrodes coated by the conductive ink. The portions of the cables on the external surface of the endotracheal tube are covered by a PVC non-conductive isolation covering.

While various embodiments have been described and illustrated, the detailed description is not to be construed as being limited hereto.

## Claims

1. A device (1) for monitoring electromyographic signals of a subject's laryngeal muscles and laryngeal nerves comprising:
- an endotracheal tube (2) extending along a longitudinal axis and having an exterior surface (20) and an internal surface (21);
- at least two electrically conductive electrodes (3) coupled to the external surface of the endotracheal tube (2), wherein the two electrically conductive electrodes are configured to receive electromyographic signals from laryngeal muscles and nerves when the endotracheal tube is placed in the trachea of the subject,
the device being **characterized in that**,
the at least two electrically conductive electrodes (3) comprise an electrically conductive layer deposited on a flexible polymer layer,
the flexible polymer layer is made of polyethylene terephthalate, polyethylene naphthalate, polycarbonate, polyurethane, polymethyl methacrylate, polyamide, or polyimide, and
the at least two electrically conductive electrodes (3) have a thickness ranging from 0.006 mm to 1.5 mm.

2. The device according to claim **1,** wherein the electrically conductive layer of each electrically conductive electrode (3) is an electrically conductive ink, notably a silver ink.

3. The device according to either one of claims **1** or **2,** further comprising an adhesive coating to couple each electrically conductive electrode (3) to the endotracheal tube (2).

4. The device according to any one of claims **1** to **3,** wherein each electrically conductive electrode (3) is placed on a deformable support (10) which is in contact with the external surface of the endotracheal tube (2), said deformable support (10) being a polymer material such as polystyrene or polyester.

5. The device according to any one of claims **1** to **4,** wherein the at least two electrically conductive electrodes (3) extending along the longitudinal axis, are laterally offset and are transversally aligned.

6. The device according to any one of claims **1** to **5,** wherein the endotracheal tube (2) comprises a distal portion to be inserted into the subject larynx and where the endotracheal tube (2) comprises an inflatable cuff (4) at said distal portion.

7. The device according to claim **6,** wherein the at least two electrically conductive electrodes (3) are spaced from the inflatable cuff (4) of about 0.2 cm to 4 cm along the longitudinal axis.

8. The device according to any one of claims **1** to **7,** further comprising at least two cables (5) coupled to the at least two electrically conductive electrodes (3) and configured to carry the electromyographic signals received by the at least two electrically conductive electrodes (3) to a processing apparatus.

9. The device according to claim **8,** wherein the endotracheal tube (2) comprises an internal channel (6) between the external surface (20) and the internal surface (21), extending along the longitudinal axis of the endotracheal tube (2) and being configured to lodge at least partially the at least two cables (5).

10. The device (1) according to any one of claims **1** to **9,** wherein the endotracheal tube (2) is a braided or reinforced endotracheal tube.

11. The device according to any one of claim **1** or **10,** comprising four, six or eight electrically conductive electrodes coupled to the external surface (20) of the endotracheal tube (2).

12. The device according to any one of claims **1** to **11,** wherein the at least two electrically conductive electrodes (3) are regularly distributed in a direction transversal to the longitudinal axis to surround a circumference of the endotracheal tube (2).

13. The device according to any one of claims **1** to **12,** wherein the distal portion of the endotracheal tube (2) comprises a Murphy's eye (8).

14. The device according to any one of claims **1** to **13,** further comprising a connector (9) configured to be mechanically connected to the proximal end of the endotracheal tube (2) to couple the endotracheal tube (2) to a mechanical ventilator.

15. The device according to claim 9, wherein each cable (5) comprises a first portion extending into the internal channel (6) and a second portion extending on the external surface of the endotracheal tube (2) in contact with one electrically conductive electrode (3).

## Patentansprüche

1. Vorrichtung (1) zur Überwachung elektromyographischer Signale der Kehlkopfmuskulatur und der Kehlkopfnerven eines Probanden, umfassend:
- einen Endotrachealtubus (2), der sich entlang einer Längsachse erstreckt und eine Außenfläche (20) und eine Innenfläche (21) aufweist;
- mindestens zwei elektrisch leitfähige Elektroden (3), die mit der Außenfläche des Endotrachealtubus (2) verbunden sind, wobei die zwei elektrisch leitfähigen Elektroden dazu konfiguriert sind, elektromyographische Signale von den Kehlkopfmuskeln und -nerven zu empfangen, wenn der Endotrachealtubus in die Luftröhre des Probanden eingeführt ist,
**dadurch gekennzeichnet, dass**
die mindestens zwei elektrisch leitfähigen Elektroden (3) eine elektrisch leitfähige Schicht umfassen, die auf einer flexiblen Polymerschicht aufgebracht ist,
die flexible Polymerschicht aus Polyethylenterephthalat, Polyethylennaphthalat, Polycarbonat, Polyurethan, Polymethylmethacrylat, Polyamid oder Polyimid besteht, und
die mindestens zwei elektrisch leitfähigen Elektroden (3) eine Dicke im Bereich von 0,006 mm bis 1,5 mm aufweisen.

2. Vorrichtung nach Anspruch 1, wobei die elektrisch leitfähige Schicht jeder elektrisch leitfähigen Elektrode (3) eine elektrisch leitfähige Tinte, insbesondere eine Silbertinte, ist.

3. Vorrichtung nach einem der Ansprüche 1 oder 2, ferner umfassend eine Klebebeschichtung zur Verbindung jeder elektrisch leitfähigen Elektrode (3) mit dem Endotrachealtubus (2).

4. Vorrichtung nach einem der Ansprüche 1 bis 3, wobei jede elektrisch leitfähige Elektrode (3) auf einer verformbaren Halterung (10) angeordnet ist, die mit der Außenfläche des Endotrachealtubus (2) in Kontakt steht, wobei die verformbare Halterung (10) aus einem Polymermaterial wie Polystyrol oder Polyester besteht.

5. Vorrichtung nach einem der Ansprüche 1 bis 4, wobei die mindestens zwei elektrisch leitfähigen Elektroden (3), die sich entlang der Längsachse erstrecken, seitlich versetzt und quer ausgerichtet sind.

6. Vorrichtung nach einem der Ansprüche 1 bis 5, wobei der Endotrachealtubus (2) einen distalen Abschnitt aufweist, der in den Kehlkopf des Probanden eingeführt werden soll, und wobei der Endotrachealtubus (2) an diesem distalen Abschnitt eine aufblasbare Manschette (4) aufweist.

7. Vorrichtung nach Anspruch 6, wobei die mindestens zwei elektrisch leitfähigen Elektroden (3) entlang der Längsachse in einem Abstand von etwa 0,2 cm bis 4 cm von der aufblasbaren Manschette (4) angeordnet sind.

8. Vorrichtung nach einem der Ansprüche 1 bis 7, ferner umfassend mindestens zwei Kabel (5), die mit den mindestens zwei elektrisch leitfähigen Elektroden (3) verbunden und dazu konfiguriert sind, die von den mindestens zwei elektrisch leitfähigen Elektroden (3) empfangenen elektromyographischen Signale an eine Verarbeitungsvorrichtung zu übertragen.

9. Vorrichtung nach Anspruch 8, wobei der Endotrachealtubus (2) einen inneren Kanal (6) zwischen der Außenfläche (20) und der Innenfläche (21) aufweist, der sich entlang der Längsachse des Endotrachealtubus (2) erstreckt und dazu konfiguriert ist, die mindestens zwei Kabel (5) zumindest teilweise aufzunehmen.

10. Vorrichtung (1) nach einem der Ansprüche 1 bis 9, wobei es sich bei dem Endotrachealtubus (2) um einen geflochtenen oder verstärkten Endotrachealtubus handelt.

11. Vorrichtung nach einem der Ansprüche 1 oder 10, umfassend vier, sechs oder acht elektrisch leitfähige Elektroden, die mit der Außenfläche (20) des Endotrachealtubus (2) verbunden sind.

12. Vorrichtung nach einem der Ansprüche 1 bis 11, wobei die mindestens zwei elektrisch leitfähigen Elektroden (3) in einer Richtung quer zur Längsachse regelmäßig verteilt sind, um einen Umfang des Endotrachealtubus (2) zu umgeben.

13. Vorrichtung nach einem der Ansprüche 1 bis 12, wobei der distale Abschnitt des Endotrachealtubus (2) ein Murphy-Auge (8) aufweist.

14. Vorrichtung nach einem der Ansprüche 1 bis 13, ferner umfassend einen Verbinder (9), der dazu konfiguriert ist, mechanisch mit dem proximalen Ende des Endotrachealtubus (2) verbunden zu werden, um den Endotrachealtubus (2) mit einem mechanischen Beatmungsgerät zu koppeln.

15. Vorrichtung nach Anspruch 9, wobei jedes Kabel (5) einen ersten Abschnitt, der sich in den inneren Kanal (6) erstreckt, und einen zweiten Abschnitt aufweist, der sich auf der Außenfläche des Endotrachealtubus (2) erstreckt und mit einer elektrisch leitfähigen Elektrode (3) in Kontakt steht.

## Revendications

1. Dispositif (1) de surveillance des signaux électromyographiques des muscles laryngés et des nerfs laryngés d'un sujet, comprenant :
- une sonde endotrachéale (2) s'étendant selon un axe longitudinal et présentant une surface extérieure (20) et une surface intérieure (21) ;
- au moins deux électrodes électriquement conductrices (3) couplées à la surface extérieure de la sonde endotrachéale (2), les deux électrodes électriquement conductrices sont configurées pour recevoir des signaux électromyographiques provenant des muscles laryngés et des nerfs lorsque la sonde endotrachéale est placée dans la trachée du sujet, le dispositif étant **caractérisé en ce que** les au moins deux électrodes électriquement conductrices (3) comprennent une couche conductrice déposée sur une couche de polymère flexible, le polymère flexible étant réalisé à partir de polyéthylène téréphtalate, de polyéthylnaphthalate, de polycarbonate, de polyuréthane, de polyméthacrylate de méthyle, de polyamide ou de polyimide, et les au moins deux électrodes électriquement conductrices ayant une épaisseur entre 0,006 mm à 1,5 mm.

2. Dispositif selon la revendication **1**, dans lequel la couche conductrice de chaque électrode conductrice (3) est une encre conductrice, notamment une encre d'argent.

3. Dispositif selon l'une quelconque des revendications **1** ou **2**, comprenant en outre un revêtement adhésif pour coupler chaque électrode conductrice (3) à la sonde endotrachéale (2).

4. Dispositif selon l'une quelconque des revendications **1** à **3**, dans lequel chaque électrode conductrice (3) est disposée sur un support déformable (10) qui est en contact avec la surface extérieure de la sonde endotrachéale (2), le support déformable étant un matériau polymère par exemple le polystyrène ou le polyester.

5. Dispositif selon l'une quelconque des revendications **1** à **4**, dans lequel lesdites au moins deux électrodes conductrices (3), qui s'étendent selon l'axe longitudinal, sont décalées latéralement et alignées transversalement.

6. Dispositif selon l'une quelconque des revendications **1** à **5**, dans lequel la sonde endotrachéale (2) comprend une portion distale destinée à être insérée dans le larynx du sujet, et où la sonde endotrachéale (2) comprend un ballonnet gonflable (4) au niveau dudit portion distale.

7. Dispositif selon la revendication **6**, dans lequel lesdites au moins deux électrodes conductrices (3) sont espacées du ballonnet gonflable (4) d'environ 0,2 cm à 4 cm le long de l'axe longitudinal.

8. Dispositif selon l'une quelconque des revendications **1** à **7**, comprenant en outre au moins deux câbles (5) couplés auxdites au moins deux électrodes conductrices (3) et configurés pour acheminer les signaux électromyographiques reçus par lesdites au moins deux électrodes conductrices (3) vers un appareil de traitement.

9. Dispositif selon la revendication **8**, dans lequel la sonde endotrachéale (2) comprend un canal interne (6) entre la surface extérieure (20) et la surface intérieure (21), s'étendant selon l'axe longitudinal de la sonde endotrachéale (2) et étant configuré pour accueillir partiellement lesdits au moins deux câbles (5).

10. Dispositif (1) selon l'une quelconque des revendications **1** à **9**, dans lequel la sonde endotrachéale (2) est une sonde endotrachéale tressée ou renforcée.

11. Dispositif selon l'une quelconque des revendications **1** ou **10**, comprenant quatre, six ou huit électrodes conductrices couplées à la surface extérieure (20) de la sonde endotrachéale (2).

12. Dispositif selon l'une quelconque des revendications **1** à **11**, dans lequel lesdites au moins deux électrodes conductrices (3) sont réparties régulièrement dans une direction transverse à l'axe longitudinal afin d'entourer la circonférence de la sonde endotrachéale (2).

13. Dispositif selon l'une quelconque des revendications **1** à **12**, dans lequel la portion distale de la sonde endotrachéale (2) comprend un œil de Murphy (8).

14. Dispositif selon l'une quelconque des revendications **1** à **13**, comprenant en outre un connecteur (9) configuré pour être connecté mécaniquement à l'extrémité proximale de la sonde endotrachéale (2) afin de coupler la sonde endotrachéale (2) à un ventilateur mécanique.

15. Dispositif selon la revendication **9**, dans lequel chaque câble (5) comprend une première partie s'étendant dans le canal interne (6) et une seconde partie s'étendant sur la surface extérieure de la sonde endotrachéale (2) en contact avec une électrode conductrice (3).
